**Europäisches Patentamt**

**European Patent Office**

**Office européen des brevets**

(19)

(11) Veröffentlichungsnummer: **0 191 013**
**A2**

(12)

# EUROPÄISCHE PATENTANMELDUNG

(21) Anmeldenummer: **86890019.2**

(22) Anmeldetag: **05.02.86**

(51) Int. Cl.⁴: **A 61 G 15/00**
**A 61 C 19/00**

(30) Priorität: **05.02.85 AT 321/85**

(43) Veröffentlichungstag der Anmeldung:
**13.08.86 Patentblatt 86/33**

(84) Benannte Vertragsstaaten:
**DE FR IT SE**

(71) Anmelder: **Kotschy, Peter, Dr.**
**Lindengasse 41/15**
**A-1070 Wien(AT)**

(72) Erfinder: **Kotschy, Peter, Dr.**
**Lindengasse 41/15**
**A-1070 Wien(AT)**

(74) Vertreter: **Boeckmann, Peter, Dipl.-Ing. et al,**
**Patentanwälte Dipl.-Ing. Peter Boeckmann, Dipl.-Ing. Leo**
**Brauneiss Strohgasse 10**
**A-1030 Wien(AT)**

(54) **Vorzugsweise fahrbar ausgebildetes Möbel für eine zahnärztliche Praxis.**

(57) An einem fahrbar ausgebildeten Möbel für eine zahnärztliche Praxis ist eine Arbeitsplatte (16) aus dem Möbelgestell (1) ausziehbar. Im Abstand (a) über dieser Arbeitsplatte (16) ist ein Werkzeugträger (17) für Kleinwerkzeuge angeordnet, der in der eingeschobenen Stellung der Arbeitsplatte (16) diese teilweise übergreift und ebenfalls aus dem Möbelgestell (1) ausziehbar ist. Der Werkzeugträger (17) läßt stets einem vorne liegenden Abschnitt der Arbeitsplatte (16) frei.

FIG.5

EP 0 191 013 A2

Vorzugsweise fahrbar ausgebildetes Möbel
für eine   zahnärztliche Praxis

Die Erfindung bezieht sich auf ein, vorzugsweise fahrbar ausgebildetes, Möbel für eine zahnärztliche Praxis, mit mehreren aus dem Möbelgestell ausziehbaren Laden zur Aufbewahrung von Dentalwerkzeugen od. dgl., sowie mit einer Arbeitsplatte.

Es sind als fahrbare Schränke ausgebildete Möbel der eingangs geschilderten Art bekannt, die aber in der Praxis nicht völlig befriedigen, insbesondere dort nicht, wo das Möbel nicht nur zum Zwecke der reinen Aufbewahrung der Dentalwerkzeuge od. dgl. verwendet werden soll, sondern auch bei Dentalarbeiten bzw. Vorarbeiten hiezu. Die bekannten fahrbaren Kästen bilden mit ihrer Deckfläche zwar eine begrenzte Arbeitsfläche, die jedoch unhandlich liegt, wenn das Möbel zum Kopfende des Behandlungsstuhles herangezogen wird. Es liegt dann entweder die Arbeitsfläche zu weit vom Kopfende weg, um handlich vom Arzt bzw. seiner Assistentin erreichbar zu sein, oder es lassen sich die Laden nicht oder nur schwierig öffnen. Dazu kommt, daß eine moderne zahnärztliche Praxis (im folgenden kurz Dentalpraxis genannt) eine Vielzahl von Arbeitsgebieten hat, so z.B. Konservierung, Operation und Vorsorgung mit Prothesen, welche Arbeitsgebiete eine Vielzahl unterschiedlicher Dentalwerkzeuge bedingen, die stets griffgerecht zur Verfügung stehen sollen. Gerade Kleinwerkzeuge, wie sie für die genannten Arbeitsgebiete laufend benötigt werden, sind mit den bekannten Möbeln nur unübersichtlich und unhandlich zur Verfügung. Zusätzliche Ablageflächen engen den Platz rund um den Behandlungsstuhl ein und erschweren die Übersicht. Die genannten Mängel treten verstärkt dort auf, wo das Möbel zwischen mehreren Behandlungsstühlen verschoben werden muß, an denen wechselweise gearbeitet wird.

Die Erfindung setzt sich zur Aufgabe, diese Nachteile zu vermeiden und ein, vorzugsweise fahrbares, Möbel für eine Dentalpraxis so zu verbessern, daß die Dentalwerkzeuge od. dgl. handlich und übersichtlich untergebracht sind, wobei zugleich ein bequemes Arbeiten am Kopfende des Patienten, also optimal ergonomisch für Arzt und Assistentin, möglich ist. Die Erfindung löst diese Aufgabe dadurch, daß bei einem Möbel der eingangs geschilderten Art die Arbeitsplatte aus dem Möbelgestell ausziehbar ist und daß im Abstand über der Arbeitsplatte ein Werkzeugträger, insbesondere für Kleinwerkzeuge, wie Schleifkörper und Bohrer, angeordnet ist, der in der eingeschobenen Stellung der Arbeitsplatte einen Bruchteil der Arbeitsplattenfläche übergreift und ebenfalls aus dem Möbelgestell ausziehbar ist, stets jedoch einen vorne liegenden Abschnitt der Arbeitsplatte unabgedeckt läßt. Auf dieser aus dem Möbelgestell herausziehbaren Arbeitsplatte läßt sich bequem in der Nähe des Kopfendes des Patienten arbeiten, ohne daß die Auszugbewegung der übrigen Laden behindert wird. Zugleich stehen die auf dem Werkzeugträger untergebrachten Kleinwerkzeuge od. dgl. übersichtlich und stets griffbereit zur Verfügung, zumal auch dieser Werkzeugträger in die jeweils gewünschte Lage aus dem Möbelgestell ausgezogen bzw. wieder zurückgeschoben werden kann. Da dieser Werkzeugträger den vorne liegenden Abschnitt der Arbeitsplatte stets freiläßt, steht dieser Arbeitsplattenabschnitt stets für Mischvorgänge oder sonstige in der Dentalpraxis übliche Arbeiten zur Verfügung, ohne den Zugriff auf die vom Werkzeugträger getragenen Kleinwerkzeuge od. dgl. zu beeinträchtigen. Der freie Raum, welcher durch den Abstand zwischen Arbeitsplatte und Werkzeugträger gebildet wird, ermöglicht es, auf der Arbeitsplatte auch im hinteren Bereich derselben Gegenstände anzuordnen, ohne daß diese vom Werkzeugträger abgestreift werden können. Stets stehen daher alle Geräte, die in der Dentalpraxis, sei es für konservierende, chirurgische, prothetische oder sonstige Behandlung benötigt werden, griffbereit zur Verfügung. Die bisher üblichen ortsfesten U-förmigen Verbauten rund um den Behandlungsstuhl können daher entfallen, wodurch der Arzt und die Assistentin mehr Bewegungsfreiheit erhalten, insbesondere wenn das Möbel verfahrbar ist. Die Verfahrbarkeit des Möbels ermöglicht es auch, die gesamten Instrumente und Geräte in den Sterilisationsraum zu verfahren, was die Sterilisationsarbeit erleichtert.

Vorzugsweise ist die Arbeitsplatte in voller Tiefe ausziehbar, wobei sie in der eingeschobenen Stellung vom Werkzeugträger etwa zur Hälfte übergriffen wird. Dadurch ist die Arbeitsplatte am besten ausnützbar.

Gemäß einer bevorzugten Ausführungsform der Erfindung hat der Werkzeugträger zumindest einen Anschlag, der hinter den hinteren Rand der Arbeitsplatte in deren Bewegungsbahn ragt. Vorzugsweise ist dieser Anschlag von einer hinteren Abschlußwand des Werkzeugträgers gebildet, wodurch ein gesonderter Anschlag, z.B. von der Seitenwand des Werkzeugträgers her, entbehrlich wird. Dieser Anschlag verhindert einerseits, daß der Werkzeugträger relativ zur Arbeitsplatte über das durch den Anschlag bestimmte Ausmaß hinaus verschoben werden kann, so daß die Arbeitsfläche der Arbeitsplatte stets freigehalten wird. Anderseits bildet dieser Anschlag eine Hilfe zum gemeinsamen Einschieben von Arbeitsplatte und Werkzeugträger, wobei die Arbeitsplatte den Anschlag des Werkzeugträgers vor sich herschiebt.

Um die Arbeitsplatte bzw. den Werkzeugträger bei der Ausziehbewegung stabil zu führen, sind im Rahmen der Erfindung Teleskopschienen vorhanden, die an sich bekannt sind. Es ist auch bekannt, an einem Ständer zur Aufnahme zahnärztlicher Werkzeuge eine Tasse verschiebbar anzuordnen (US-PS 4 206 546). Weiters sind Vorrichtungen zur Aufnahme zahnärztlicher Gerätschaften in Form von auf schwenkbaren Armen angeordneten Tassen oder Kästen bekannt (DE-OS 3 302 189, US-PS 4 209 908). Mit diesen bekannten Ausbildungen lassen sich die erfindungsgemäßen Vorteile aber nicht erzielen.

Gemäß einer Weiterbildung der Erfindung ist der Werkzeugträger mit der Arbeitsplatte auf kraftbegrenzte Mitnahme bei der Auszugbewegung verbunden. Dadurch ist es möglich, den Werkzeugträger bei der Auszugbewegung der Arbeitsplatte mitzunehmen, ihn jedoch von der Kupplung mit der Arbeitsplatte jederzeit durch leichten Druck, welcher die Mitnahmekraft überwindet, lösen zu können. Geeignete Beschläge für eine solche begrenzte Mitnahme sind an sich bekannt, z.B. federbelastete Kugelrasten oder gebremste Teleskopschienen.

Gemäß einer vorteilhaften Weiterbildung der Erfindung sind die Arbeitsplatte und der Werkzeugträger in der eingeschobenen Stellung durch einen Klappdeckel des Möbelgestelles abdeckbar, der in seiner Öffnungsstellung eine horizontalliegende Arbeitsfläche hat. In seiner Geschlossenstellung schirmt der Klappdeckel den Werkzeugträger und die von ihm getragenen, oft empfindlichen Kleinwerkzeuge gegen Berührung und Verschmutzung ab, ebenso die Arbeitsplatte. Die zusätzliche Arbeitsfläche, welche der Klappdeckel mit seiner in der Offenstellung obenliegenden Innenfläche bildet, ist vorteilhaft, z.B. bei der Durchführung von Anrührvorgängen od. dgl., aber auch als zusätzliche Abstellfläche usw.

Da die Arbeitsplatte in der ausgezogenen Stellung, insbesondere bei Vollauszug, verhältnismäßig weit aus dem Möbelgestell auskragt, wird bei starkem vertikalen Druck auf die Arbeitsplatte ein beträchtliches Kippmoment auf das Möbel ausgeübt. Zwar hat das Möbel, insbesondere bei durch schwerere Werkzeuge, wie Zangen beladenen, eingeschobenen Laden, eine beträchtliche Standfestigkeit, es ist jedoch vorteilhaft, wenn im Möbelgestell an der der Auszugstellung der Arbeitsplatte entgegengesetzten Seite ein Gegengewicht, z.B. zumindest eine Stahlplatte, eingebaut ist. Dieses Gegengewicht wirkt dem Kippmoment entgegen, so daß Unfällen vorgebeugt wird. Zweckmäßig ist dieses Gegengewicht in der Bodenplatte des Möbelgestelles angeordnet, um den Gesamtschwerpunkt möglichst tief zu halten.

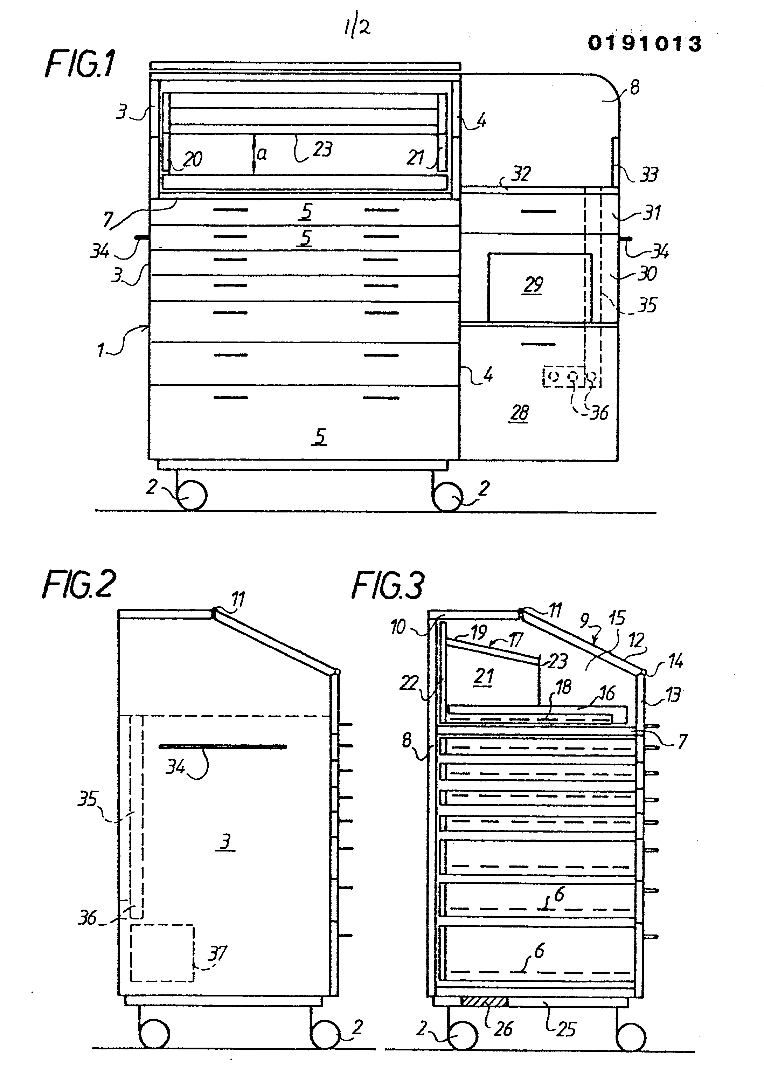

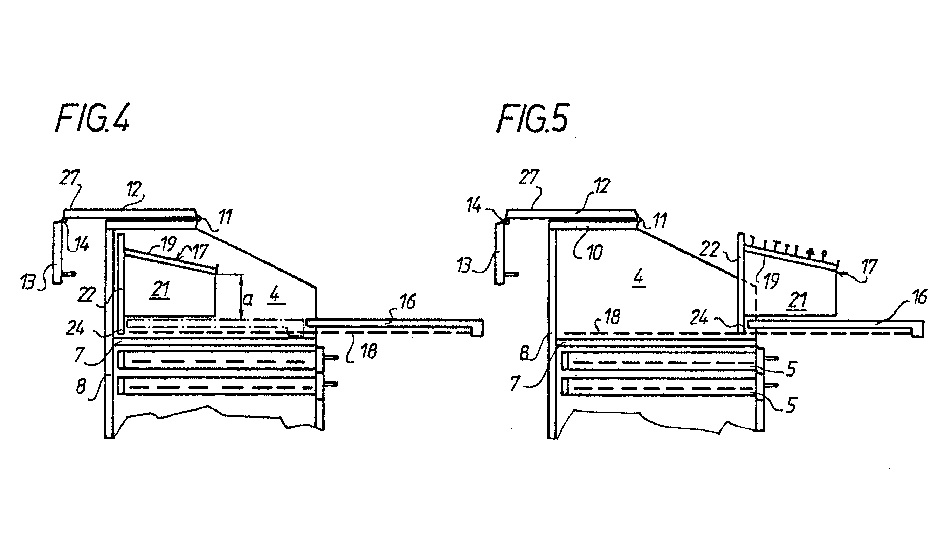

In der Zeichnung ist der Erfindungsgegenstand an Hand eines Ausführungsbeispieles schematisch veranschaulicht. Fig. 1 zeigt das Möbel bei geöffnetem Klappdeckel in Vorderansicht, Fig. 2 bei geschlossenem Klappdeckel in Seitenananansicht. Fig. 3 ist ein Vertikalschnitt durch das Möbel bei geschlossenem Klappdeckel. Die Fig. 4 und 5 zeigen zwei mögliche Auszugstellungen der Arbeitsplatte bzw. des Werkzeugträgers im Schnitt ähnlich Fig. 3.

Das Möbel hat ein Möbelgestell 1, z.B. aus Holz, Kunststoff oder Metall, insbesondere Stahlblech, das auf Rollen 2 verfahrbar ist. Das Möbelgestell 1 hat zwei seitliche Häupter 3, 4, an denen mittels üblicher Auszugsführungen, insbesondere Teleskopschienen 6, mehrere Laden 5 geführt sind, deren jede mit einer Einrichtung zur Aufnahme von Instrumenten bzw. Materialien für die Dentalpraxis versehen ist. Oberhalb der obersten Lade 5 sind die beiden Häupter 3, 4 durch eine horizontale Zwischenplatte 7 verbunden, die an die Rückwand 8 (Fig. 3) des Möbels dicht anschließt. Die Rückwand 8 sowie die beiden Häupter 3, 4 sind über diese Zwischenplatte 7 nach oben verlängert und tragen eine horizontale Deckplatte 10, an der mittels eines Scharnieres 11 ein zweiteiliger Klappdeckel 9 schwenkbar befestigt ist, dessen beide Teile 12, 13 untereinander durch ein Scharnier 14 mit horizontaler Achse verbunden sind. Durch diesen Klappdeckel 9 ist der oberhalb der Zwischenplatte 7 liegende Raum 15 dicht abschließbar. In diesem Raum 15 finden eine ausziehbare Arbeitsplatte 16 sowie ein gleichfalls ausziehbarer Werkzeugträger 17 in ihrer in das Möbelgestell 1 eingeschobenen Stellung Platz. Die Arbeitsplatte 16 ist mittels üblicher Teleskopschienen 18 aus der in Fig. 3 dargestellten, in das Möbelgestell 1 eingeschobenen Stellung in eine in Fig. 4 dargestellte, vollausgezogene Stellung ausziehbar, wobei jede Zwischenstellung möglich ist. In ähnlicher Weise ist der Werkzeugträger 17 aus der in Fig. 3 dargestellten, voll eingeschobenen Stellung in die in Fig. 5 dargestellte, voll ausgezogene Stellung ausziehbar, wobei gleichfalls jedwede Zwischenstellung möglich ist. Der Werkzeugträger 17 besteht aus einer geringfügig geneigten Platte 19, die zwischen zwei seitlichen vertikalen Wangen 20, 21 angeordnet und an eine dieser beiden Wangen 20, 21 verbindende vertikale hintere Abschlußwand 22 angesetzt ist. Die Platte 19 befindet sich mit ihrer tiefer liegenden Vorderkante 23 im Abstand a (Fig. 1) über der Oberfläche der Arbeitsplatte 16, so daß auf dieser für die Zahnarztpraxis benötigte Materialien stehen können, ohne am Werkzeugträger 17 anzustreifen. Der Werkzeugträger 17 ist an der Arbeitsplatte 16 bzw. an den beiden Häuptern 3, 4 mittels nicht dargestellter Teleskopschienen zwischen den in den Fig. 3 und 5 dargestellten Grenzlagen verschiebbar geführt. Im Prinzip ist der Werkzeugträger 17 von der Arbeitsplatte 16 unabhängig verschiebbar, jedoch ist die Anordnung so getroffen, daß der Werkzeugträger 17 die Arbeitsplatte 16 maximal bis etwa zur Hälfte übergreifen kann. Hiezu ist die hintere Abschlußwand 22 des Werkzeugträgers 17 nach unten verlängert und bildet einen Anschlag 24, der am Hinterrand der Arbeitsplatte 16 zur Anlage kommt, sobald der Werkzeugträger 17 die Arbeitsplatte 16 im maximal zulässigen Maß abdeckt. Zugleich sichert dieser Anschlag 24 die Mitnahme des Werkzeugträgers 17 beim Zurückschieben der Arbeitsplatte 16 in die eingeschobene Stellung (Fig. 3). Um auch bei der Auszugbewegung der Arbeitsplatte 16 den Werkzeugträger 17 mitzunehmen, ist letzterer mit der Arbeitsplatte 16 auf kraftbegrenzte Mitnahme verbunden, d.h. die Mitnahme des Werkzeugträgers 17 erfolgt, solange auf ihn nicht eine vorbestimmte Bremskraft ausgeübt wird. Hiezu können übliche Bauteile dienen, z.B. gebremste Teleskopschienen oder in die Arbeitsplatte 16 bzw. in die seitlichen Wangen 20, 21 des Werkzeugträgers 17 eingebaute federbelastete Kugelschnapper, deren Kugeln in entsprechende Rasten des jeweils anderen der Bauteile 16, 17 einrasten, wobei diese Verbindung durch mit vorbestimmter Kraft erfolgendes Auseinanderziehen der beiden Bauteile 16, 17 lösbar ist, durch Gegeneinanderschieben der beiden Bauteile aber leicht wieder zum Einrasten zu bringen ist.

Auf dem einen fahrbaren Ständer bildenden Werkzeugträger 17 können die für die verschiedensten Behandlungen der Dentalpraxis erforderlichen Kleinwerkzeuge, insbesondere Schleifkörper, Bohrer usw. platzsparend, übersichtlich und griffbereit angeordnet werden, was in Fig. 5 für einige dieser Werkzeuge schematisch angedeutet ist. Die nach oben etwas über den oberen hinteren Rand der Platte 19 vorragende Abschlußwand 22 bildet einen Schutz für diese Kleinwerkzeuge bei der Einschubbewegung, so daß diese Werkzeuge nicht mit der Deckplatte 10 kollidieren können. Die in die jeweils gewünschte Lage ausziehbare Arbeitsplatte 16 ermöglicht es zusammen mit dem ebenfalls in die jeweils gewünschte Lage ausziehbaren Werkzeugträger 17, Arbeitsplatte und Werkzeuge in guter Sicht und in handlicher Reichweite vom Stuhl des Arztes bzw. der Assistentin anzuordnen, so daß bei größtmöglicher Bequemlichkeit und Handlichkeit stets ein optimales Platzangebot zur Arbeit zur Verfügung steht.

Auf der Arbeitsplatte 16 können beliebige Arbeitsvorgänge durchgeführt werden. Da es hiebei denkbar ist, daß auf die Arbeitsplatte 16 Druck von oben und dadurch auf das Möbel ein Kippmoment ausgeübt wird, ist in einer Bodenplatte 25 ein Gegengewicht 26, z.B. in Form von Stahlplatten, angeordnet, welches auf der der ausgezogenen Stellung der Arbeitsplatte 16 gegenüberliegenden Seite des Gesamtschwerpunktes des Möbels liegt und daher dem vom Druck auf die Arbeitsplatte 16 ausgeübten Kippmoment entgegenwirkt.

Der Klappdeckel 9 läßt sich in die in den Fig. 4 und 5 dargestellte Offenstellung zurückklappen, in welcher das größere der beiden Teile 12, 13, nämlich das Teil 12, mit seiner nun oben liegenden Innenfläche 27 eine horizontal liegende zusätzliche Arbeitsfläche bildet, auf der z.B. Mischvorgänge od. dgl. durchgeführt werden können.

Das Möbel kann, wie in Fig. 1 gezeigt, mit an das Haupt 4 seitlich angebauten Anbauten versehen sein, z.B. mit einem Auszug 28 für einen Abfallkorb, in welchen der Abfall durch eine Einwurfklappe 29 eingeworfen werden kann, die in eine Türe 30 eingebaut ist. Oberhalb der Türe 30 kann eine zusätzliche Lade 31 vorgesehen sein, oberhalb welcher eine Abstellplatte 32, z.B. für Mischgeräte, angeordnet sein kann. Diese Bauteile können seitlich durch ein zusätzliches Haupt 33 untereinander versteift sein. In dieser Abstellplatte 32, die etwa auf gleicher Höhe liegt wie die ausziehbare Arbeitsplatte 16, befindet sich im Bereich der Rückwand 8 eine mit einer Gummidichtung versehene Klappe, durch welche ein Kabelschacht 35 (Fig. 1, 2) zugänglich ist, der zu mehreren an der Rückwand 8 des Möbelgestelles 1 versenkt angeordneten Steckdosen 36 führt. Diese Steckdosen werden von einem Stromkabel mit elektrischer Energie versorgt, das auf eine Kabelrolle mit automatischem Kabeleinzug aufgerollt ist, die in einem durch eine Klappe 37 (Fig. 2) zugänglichen Fach an der vom Haupt 33 gebildeten Seitenwand des Möbelgestelles 1 angeordnet ist. Dadurch können elektrisch angetriebene Hilfsgeräte der Dentalpraxis, wie Amalgammischer, Photolampen oder photochemische Geräte, die auf der Abstellplatte 32 angeordnet sind, betrieben werden, ohne daß die Stromkabel außerhalb des Möbelgestelles verlaufen, wodurch die Sicherheit erhöht wird.

Zur Erleichterung des Verfahrens des Möbels sind an den seitlichen Häuptern 3, 33 Griffstangen 34 vorgesehen.

0191013

- 1 -

Patentansprüche:

1. Vorzugsweise fahrbar ausgebildetes Möbel für eine zahnärztliche Praxis, mit mehreren aus dem Möbelgestell ausziehbaren Laden zur Aufbewahrung von Dentalwerkzeugen od. dgl., sowie mit einer Arbeitsplatte, dadurch gekennzeichnet, daß die Arbeitsplatte (16) aus dem Möbelgestell (1) ausziehbar ist und daß im Abstand (a) über der Arbeitsplatte (16) ein Werkzeugträger (17) angeordnet ist, der in der eingeschobenen Stellung der Arbeitsplatte (16) einen Bruchteil der Arbeitsplattenfläche übergreift und ebenfalls aus dem Möbelgestell (1) ausziehbar ist, stets jedoch einen vorne liegenden Abschnitt der Arbeitsplatte (16) unabgedeckt läßt.

2. Möbel nach Anspruch 1, dadurch gekennzeichnet, daß der Werkzeugträger zur Aufnahme von Kleinwerkzeugen, wie Schleifkörper und Bohrer, eingerichtet ist.

3. Möbel nach Anspruch 1 oder 2, dadurch gekennzeichnet, daß die Arbeitsplatte (16) in voller Tiefe ausziehbar ist und in der eingeschobenen Stellung vom Werkzeugträger (17) etwa zur Hälfte übergriffen wird.

4. Möbel nach Anspruch 1, 2 oder 3, dadurch gekennzeichnet, daß der Werkzeugträger (17) zumindest einen Anschlag (24) hat, der hinter den hinteren Rand der Arbeitsplatte (16) in deren Bewegungsbahn ragt.

5. Möbel nach Anspruch 4, dadurch gekennzeichnet, daß der Anschlag (24) von einer hinteren Abschlußwand (22) des Werkzeugträgers (17) gebildet ist.

6. Möbel nach einem der Ansprüche 1 bis 5, dadurch gekennzeichnet, daß Teleskopschienen (18) für die Auszugbewegung der Arbeitsplatte (16) bzw. des Werkzeugträgers (17) vorhanden sind.

7. Möbel nach einem der Ansprüche 1 bis 6, dadurch gekennzeichnet, daß der Werkzeugträger (17) mit der Arbeitsplatte (16) auf kraftbegrenzte Mitnahme bei der Auszugbewegung verbunden ist, z.B. mittels gebremster Teleskopschienen oder mittels federbelasteter Kugelrasten.

8. Möbel nach einem der Ansprüche 1 bis 7, dadurch gekennzeichnet, daß die Arbeitsplatte (16) und der Werkzeugträger (17) in der eingeschobenen Stellung durch einen Klappdeckel (9) des Möbelgestelles (1) abdeckbar sind, der in seiner Öffnungsstellung eine horizontal liegende Arbeitsfläche (27) hat.

9. Möbel nach einem der Ansprüche 1 bis 8, dadurch gekennzeichnet, daß im Möbelgestell (1) an der der Auszugstellung der Arbeitsplatte (16) entgegengesetzten Seite ein Gegengewicht (26), z.B. zumindest eine Stahlplatte, eingebaut ist.

10. Möbel nach Anspruch 9, dadurch gekennzeichnet, daß das Gegengewicht in einer Bodenplatte (25) des Möbelgestelles (1) angeordnet ist.

11. Möbel nach einem der Ansprüche 1 bis 10, dadurch gekennzeichnet, daß zu einer neben der ausziehbaren Arbeitsplatte (16) angeordneten, gestellfesten Abstellplatte (32) ein Kabelschacht (35) führt, der diese Abstellplatte (32) mit zumindest einer am Möbelgestell (1) angeordneten Steckdose (36) verbindet.

12. Möbel nach Anspruch 11, dadurch gekennzeichnet, daß der Kabelschacht (35) an der Rückwand (8) des Möbelgestelles (1) angeordnet ist und zu einer am Möbelgestell (1) versenkt angeordneten Steckdose (36) führt.

0191013

FIG.1

FIG.2

FIG.3

FIG.4

FIG.5